# EUROPEAN PATENT SPECIFICATION

(11) **EP 2 854 864 B1**
(45) Date of publication and mention of the grant of the patent: **04.05.2016**
(21) Application number: 13742177.2
(22) Date of filing: 10.07.2013
(51) Int. Cl.: A61K 47/48, A61P 35/00

(54) **COMPOSITIONS COMPRISING OLIGOMERS OF GEMCITABINE FOR USE IN THERAPY**
ZUSAMMENSETZUNG ENTHALTEND OLIGOMERE VON GEMCITABIN ZUR THERAPEUTISCHEN ANWENDUNG
COMPOSITIONS CONTENANT DES OLIGOMÈRES DU GEMCITABINE DESTINÉES AU TRAITEMENT THÉRAPEUTIQUE

(43) Date of publication of application: 08.04.2015
(73) Proprietor: Asteriapharma GmbH, 68723 Oftersheim (DE)
(72) Inventor: JUAREZ GUERRA, Victoria, 69126 Heidelberg (DE)
(74) Representative: Patent- und Rechtsanwälte Ullrich & Naumann
(86) International application number: PCT/EP2013/064604
(87) International publication number: WO 2015/003747

(56) References cited:
- US-A- 5 614 505
- LIU J ET AL: "Increased cytotoxicity and decreased in vivo toxicity of FdUMP[10] relative to 5-FU", NUCLEOSIDES & NUCLEOTIDES, MARCEL DEKKER INC, US, vol. 18, no. 8, 1 January 1999 (1999-01-01), pages 1789-1802, XP002178372, ISSN: 0732-8311
- LIK VOON KIEW ET AL: "Efficacy of a Poly-L-Glutamic Acid-Gemcitabine Conjugate in Tumor-Bearing Mice", DRUG DEVELOPMENT RESEARCH, vol. 73, no. 3, 1 May 2012 (2012-05-01), pages 120-129, XP055100985, ISSN: 0272-4391, DOI: 10.1002/ddr.21012
- DHARMIKA S P LANSAKARA-P ET AL: "Synthesis and evaluation of novel lipophilic monophosphorylated gemcitabine derivatives and their nanoparticles", INTERNATIONAL JOURNAL OF PHARMACEUTICS, ELSEVIER BV, NL, vol. 429, no. 1, 7 March 2012 (2012-03-07) , pages 123-134, XP028406723, ISSN: 0378-5173, DOI: 10.1016/J.IJPHARM.2012.03.014 [retrieved on 2012-03-16]

## Description

The present invention relates to homo-oligomeric derivatives of gemeitabine (dFdC), a cytidine antimetabolite, for use in therapy.

### BACKGROUND OF THE INVENTION

2'.2'-difluoro 2'-deoxycytidine (abbreviated dFdC), also known as gemcitabine, is a synthetic analog of the natural nucleoside deoxycitidine. After uptake through the cell membrane by nucleoside transporters, gemcitabine is converted intracellularly first to difluorodeoxycytidine monophosphate (dFdCMP) by the enzyme deoxycitidine kinase, and subsequently to its active diphosphate (dFdCDP) and triphosphate (dFdCTP) metabolites. During S-phase dFdCTP is incorporated to the nascent DNA chain and this leads to premature chain termination, after addition of one more nucleotide, since the proofreading enzymes are not able to remove dF-cytosine and the DNA polymerases cannot continue the synthesis. Furthermore, the dFdCDP acts as an inhibitor of the enzyme ribonucleotide reductase (RR). RR is the only known enzyme that catalyzes conversion of ribonucleotides to deoxyribonucleotides, and thus it maintains the deoxynucleotide triphosphate (dNTP) pool. Therefore inhibition of RR results in reduction of the dCTP pool, which favors the incorporation of dFdCDP in the newly synthesized DNA. The human RR has three subunits: one large (RRM1) and two small (RRM2 and p53R2) polypeptides. The catalytic form of RR consists of two copies of the large subunit and one copy of each small subunit.

It has been postulated that dFdCDP binds irreversibly to RRM1 and causes its inactivation (Davidson et al., 2004). Further, it has been shown that RRM1 is up-regulated in pancreatic cancer cells resistant to dFdC when compared to the parental cells. In addition, it has been shown a significant correlation between high RRM1 levels and poor survival in pancreatic cancer patients treated with gemcitabine (Nakahira et al, 2007).

Inactivation of dFdC may also be due to the enzyme cytidine deaminase, which converts it to 2,2'-difluorodeoxyuridine (dFdU). In addition, the enzyme 5'-nucleotidase catalyses conversion of nucleotides to nucleosides, thus counteracting the action of nucleoside kinases.

Following intravenous administration, dFdC has a short half-life in the human plasma, about 8-17 minutes (Abbruzzese et al, 1991; Reid et al, J 2004), probably because it is rapidly degraded by deaminase in the blood. In tissues, its half-life seems to be longer. It has been shown in mice that radiolabeled dFdC remains up to 3 hours in normal tissues and sarcoma tissue implanted subcutaneously, although the rate of conversion to dFdU was not measured in those studies (Moog et al, 2002; Shipley et al, 1992). It is excreted in the urine mainly as its primary metabolite, dFdU, and only less than 10% is excreted unprocessed (Noble S, Goa KL, 1997; Moog et al, 2002). Thus, only a small amount of dFdC can reach the tumor cells and can be converted into its active dFdCDP and dFdCTP derivatives. This could be one reason why, although gemcitabine exhibits in vitro a strong cytotoxic activity comparable to that of other anticancer drugs such as doxorubicin, for the treatment of cancer patients it has to be administered at the high dose of 1000 mg/m² of body surface. Such high dose increases the risk of dose-dependent side effects, most notably myelosuppression and hepatic toxicity. Thus, rapid degradation of dFdC in serum, liver and in the tumor tissue by the enzyme cytidine deaminase, which removes the amino group at position 4 of the base, represents an important obstacle for the efficacy of dFdC. In addition, another reason for the need of administrating high doses is that only a fraction of administered dFdC becomes activated by phosphorylation.

Presently, dFdC is the standard chemotherapeutic drug used in the therapy of pancreatic cancer. However, the five-year survival rate of this cancer remains below 5% due to relapse and resistance to dFdC. Therefore, improvements in the chemotherapy based on dFdC are urgently needed.

To date, attempts to overcome the aforementioned problems have failed. Recently, Sampath et al (2010) have described a method based on heteropolymeric compounds comprising long chains of nucleosides or nucleoside analogs, including dFdC, linked by a phosphodiester group. The drawbacks of Sampath's method are twofold. First, the efficacy of heteropolymeric prodrug molecules has not been demonstrated, the main problem being that the active compounds (nucleoside and nucleoside analogs) are linked by nuclease resistant moieties according to the method described by Sampath. Second, no efficient methods are currently available to deliver such long polymeric molecules to cells in vivo. Sampath et al did not report that this method works neither in vitro nor in vivo. In an earlier report, Gmeiner et al described homo-oligonucleotides consisting of monomers of 5-fluoruridine 5'-monophosphate (5-FUMP) or 5-fluodeoxyuridine 5'-monophosphate (5-FdUMP) covalently linked via phosphodiester linkages. The problems of this method are the rapid degradation of the polymers, which may be due to the inherent instability of the compounds used, and the in vivo toxicity. Liu et al describe tests of the efficacy of cancer treatment of 5-Fluorouracil (5-FU). In particular data is presented indicating that the 10mer FdUMP[10] is more cytotoxic than 5-FU towards human colorectal tumor cells (H630) and also has a decreased toxicity in vivo administrated to balb-C mice compared to 5-FU (Liu et al., 1999). US 5,614,505 describes the synthesis of homo-oligomeric 5-Fluorouritdine and 5-Fluorodeoxyuridine and the uses thereof as a polymeric drug delivery system for production of FdUMP.

Furthermore, several strategies have been tried to overcome the rapid degradation of dFdC and improve its stability, bioavailability and anti-tumor activity. Liposomes have been used to protect gemcitabine from degradation. Encapsulation of dFdC into liposomes can be easily achieved, as it is a small molecule, which can diffuse through the liposomal layer (15). Indeed, dFdC has been encapsulated into vesicular phospholipid gel, which showed increased concentration in plasma compared with free dFdC (Moog et al, 2002; Bornmann et al, 2008). However, at physiologic pH dFdC is uncharged and diffuses through the liposomal membrane. In addition, dFdC appears to induce degradation of phospholipids in the liposomal bilayer (Moog et al, 2000). Thus, liposomal dFdC showed increased toxicity, probably due to the uncontrolled release of the drug from the liposomes.

The technical problem underlying the present application could in the broadest sense be seen as dealing with the drawbacks of the prior art. Specifically, the technical problem could be viewed as overcoming low efficacy and resistance of mammalian subjects to dFdC therapy.

The present invention solves the problem of low efficacy of and resistance to dFdC therapy by providing compounds and formulations that increase the efficacy of dFdC in the treatment of patients in need thereof with new compounds and formulations of homo-oligomeric dFdC, which are disclosed in this invention.

### BRIEF SUMMARY OF THE INVENTION

Although dFdC has a strong cytotoxic activity in vitro, it has to be administered to cancer patients at high doses (above 1000 mg/m² per dose), a fact that increases the risk of side effects. In addition, such doses appear to favor the selection of highly resistant clones within the tumor tissue, which are very difficult and often impossible to remove, since the dose of dFdC cannot be increased further.

This invention relates to a compound made of 2-30 units of dFdC linked with 5'-3' phosphate bonds for use in therapy. The invention further relates to a composition comprising such a compound for use in therapy.

These compounds and compositions that help overcome those problems by combining (i.e. making) molecules of dFdC in oligomeric form. Such oligomeric forms are preferably homomeric. The term "oligomer" when used herein includes 2-mers, 3-mers, 4-mers, 5-mers, 6-mers, 7-mers, 8-mers, 9-mers, 10-mers, 15-mers, 20-mers, 25-mers or 30-mers of dFdC. A particularly preferred 3-mer is a FpFpFp trinucleotide. Such compositions comprising the compounds of the present invention have, in vitro and in vivo, superior cytotoxic effects at lower doses than dFdC and are effective against tumor cells resistant to this drug. Therefore, they represent a novel anticancer and antiviral agent.

The present invention provides compounds and compositionsuseful for treating cancer, including the following types of neoplasia: neoplasia of the exocrine pancreas, esophagus, stomach, gallbladder, liver, small intestine, vermiform appendix, anus, peritoneum, adenocarcinoma of the colon and rectum; neoplasia of the cervix (cervical intraepithelial neoplasia through invasive cervical cancer), endometrial adenocarcinoma, ovarian cancer, neoplasia of the vulva and vagina, gynecologic sarcomas; neoplasias of the breast; acute and chronic myeloid leukemias, acute and chronic lymphocytic leukemias, hairy cell leukemia, mast cell leukemia, myelodysplastic syndromes, HTLV-1 and T-cell leukemia/lymphoma, Hodgkin's disease, non-Hodgkin lymphomas; renal cell carcinoma, neoplasia of the renal pelvis, ureter, bladder, urethra, prostate, testis, penis; neoplasias of the pituitary, thyroid, adrenal cortex, neuroendocrine and enteropancreatic endocrine system; neuronal neoplasias, medulloblastoma, schwannoma, meningioma, meningeal sarcoma, glioblastoma multiforme, astrocytoma, pineal, oligodendroglial, ependymal and choroid plexus neoplasias; basal cell carcinoma, squamous cell carcinoma, melanoma, dermatofibrosarcoma, Merkel cell tumor, rhabdomyosarcoma, retinoblastoma; head and neck cancer, odontogenic tumors, neoplasias of the oral cavity, pharynx, larynx; large cell lung carcinoma, small cell lung carcinoma, non-small cell lung carcinoma, neoplasias of the thorax, malignant mesothelioma, thymomas; osteogenic sarcoma, malignant fibrous histiocytoma, chondrosarcoma, Ewing's sarcoma, primitive neuroectodermal tumor, angiosarcoma, and Kaposi's sarcoma.

The following disorders are preferred for treatment with the compositions of this invention: pancreatic cancer, hepatocellular cancer, lung cancer, cervical precancer and cancer lesions, breast cancer, ovarian cancer, colorectal cancer and lymphoma, such as non-Hodgkin's lymphoma. The compositions of the present invention are especially useful for the treatment of pancreatic cancer.

The present invention relates to compounds comprising 2-30 dFdC units linked with 5'-3' phosphate bonds for use in therapy, in particular the treatment of infectious diseases or neoplasia.

The present invention also relates to compounds comprising 2-30 dFdC units linked with 5'-3' phosphate bonds, in particular dFdC homo-oligomers, and another anticancer agent for use in therapy, in particular the treatment of infectious diseases or neoplasia comprising separate or simultaneous administration.

In a further embodiment, the present invention relates to compounds comprising 2-30 dFdC units linked with 5'-3' phosphate bonds, in particular dFdC homo-oligomers, conjugated to a targeting peptide, aptamer or therapeutic antibody for use in the treatment of an infectious disease or neoplasia..

In yet another embodiment of compounds for use according to the present invention, the use comprises the separate or simultaneous administration of the compounds according to the present invention, in particular dFdC homo-oligomers, conjugated to one or more targeting peptides or therapeutic antibodies and an anticancer agent.

In another embodiment, the present invention relates to pharmaceutical compositions comprising the compounds comprising 2-30 dFdC units linked with 5'-3' phosphate bonds, in particular dFdC homo-oligomers, with or without targeting peptides, amino acids or liposomal encapsulation, and therapeutic antibodies for use in therapy, for administration simultaneously or sequentially. The dFdC homo-oligomers according to the invention may also be encapsulated in a liposome.

The compositions of the present invention comprise the compounds as described herein. These compounds are made of 2-30 units of dFdC linked with 5'-3' phosphate bonds for use in therapy. Preferably, these compounds are made of 2-10 units dFdC linked with 5'-3' phosphate bonds for use in therapy. More preferably, the compound is FpFpF.

### DETAILED DESCRIPTION OF THE INVENTION

The preferred purpose of this invention is to provide compositions, compounds and pharmaceutical compositions containing the FpFpF trinucleotide (also referred herein as dFdC trinucleotide) as the main active ingredient, as well as specify therapeutically effective dosages resulting as effective as, or more effective than dFdC in therapy.

Active Compounds:
In one embodiment a compound of structural Formula (I) is provided: 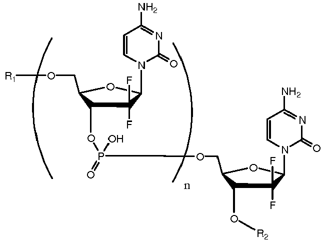 or a pharmaceutically acceptable salt, hydrate, N-oxide or solvate thereof, wherein:
   R₁ is hydrogen, lipid, amino acid, peptide, antibody or aptamer
   R₂ is hydrogen, lipid, amino acid, peptide, antibody or aptamer
   n= 1-30
   TpF: thymidinyl 3'-5' phosphate 2'-deoxy-2',2'-difluorocytidine
   FpF: 2'-deoxy-2',2'-difluorocytidinyl 3'-5' phosphate 2'-deoxy-2',2'-difluorocytidine (C₁₈H₂₁O₁₀N₆F₄P)
   FpFpF: 2'-deoxy-2',2'-difluorocytidinyl 3'-5'phosphate 2'-deoxy-2',2'-difluorocytidinyl 3'-5' phosphate-2'-deoxy-2',2'-difluorocytidine (C₂₇H₃₁O₁₆N₉F₆P₂; pangemine™)
   5'-FpFpF-3'-palmitoyl: 2'-deoxy-2',2'-difluorocytidinyl 3'-5' phosphate 2'-deoxy-2',2'-difluorocytidinyl 3'-5' phosphate-2'-deoxy-2',2'-difluorocytidinyl 3'-5' phosphate-1-α(6-(palmitoylamino)hexyl)-2-deoxy-D-ribose

Thus, apart from the preferred FpFpF trinucleotide, the present invention also provides compositions, compounds and pharmaceutical compositions containing dFdC oligomers as described and defined herein as the main active ingredient, as well as specify therapeutically effective dosages resulting as effective as, or more effective than dFdC in therapy.

### Pharmaceutical compositions

The compounds of this invention can be applied therapeutically in an acceptable pharmaceutical composition. They can be used in combination with standard aqueous and non-aqueous vehicles known to those skilled in the art, including sterile water, saline, salts obtained by addition of inorganic or organic acids such as hydrochloric acid and lactic acid, and by addition of inorganic or organic bases like sodium hydroxide and substituted ethanolamine, buffered solutions at physiological pH, Ringer's dextrose, electrolyte replenishers, thickeners, carriers, surfactants, non-aqueous solvents such as polyethylene glycol, ethyl oleate, alcoholic solutions, preservatives and other additives such as chelating agents, antioxidants and antimicrobials.

### Dosages

The dosage should be decided according to the disease to be treated and the clinical history of the patient. Thus, typically the dosage will vary with the age, gender and clinical condition of the patient and should be adjusted by the skilled expert physician. The dosage recommended can be in the range from 0.01 mg/Kg to 100 mg/Kg, preferably in the range 500-1000 mg/m², in one or more administrations in periods of one or more days, preferably using fixed dose rate by intravenous infusion. However, the dosages used may need to be adjusted according to the toxicological and pharmacokinetic parameters of each particular patient and compound, and to whether the compound is administered alone or in combination with other drugs or if a drug delivery system is used.

### Subjects

The compounds and compositions of the present invention are applied for the treatment or amelioration of the diseases as described herein of mammals, preferably humans.

### Uses

The compounds and compositions for use in therapy as described herein, may be used for treatment of diseases of mammals, preferably humans. According to one embodiment of the compounds and compositions for the use, the use comprises an administration of a therapeutically effective amount of a composition or compound of the present invention to a mammal, preferably human in need thereof.

### FIGURES

**Figure 1****.** Inhibition of proliferation of human cancer cells treated with either dFdC or homo-oligomers and hetero-oligomers containing dFdC. HeLa cells growing on 96-well plates (5000 cells/well) were treated with: 1) TpF; 2) FpF; 3) TpFpF; 4) FpFpF; 5) dFdC. The concentration of drug in the culture medium was 20 mM. Cell proliferation was measured 5 days after treatment was started using the WST-1 assay. The mean value obtained with untreated cells was set at 1.
**Figure 2****.** Proliferation of MIA PaCa-2 cells untreated (0) or treated with either dFdC or FpFpF trinucleotidde at the indicated concentrations.

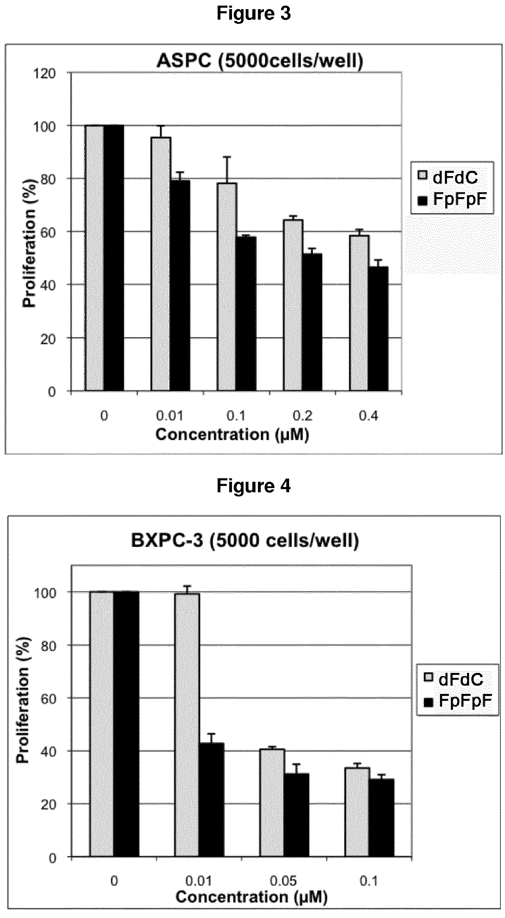

**Figure 3****.** Proliferation of ASPC cells untreated (0) or treated with either dFdC or FpFpF trinucleotide at the indicated concentrations.
**Figure 4****.** Proliferation of BXPC-3 cells untreated (0) or treated with either dFdC or FpFpF trinucleotide at the indicated concentrations.

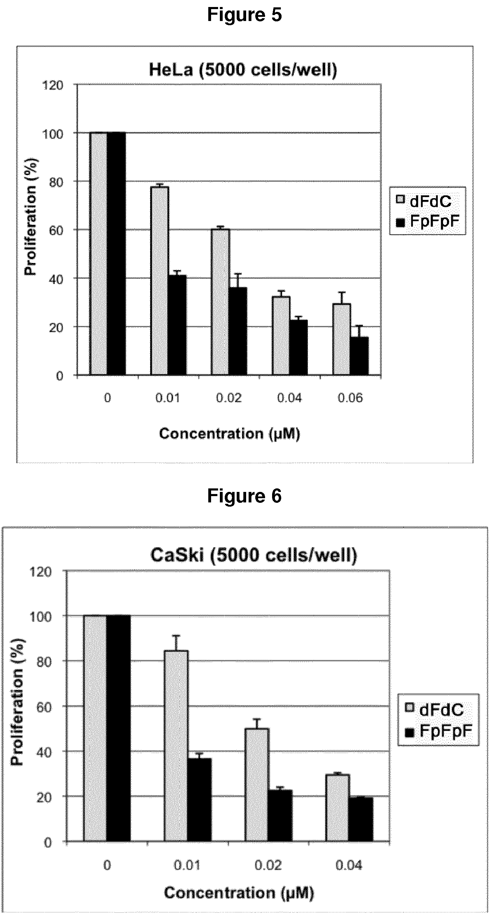

**Figure 5****.** Proliferation of HeLa cells untreated (0) or treated with either dFdC or FpFpF trinucleotide at the indicated concentrations.
**Figure 6****.** Proliferation of CaSki cells untreated (0) or treated with either dFdC or FpFpF trinucleotide at the indicated concentrations.

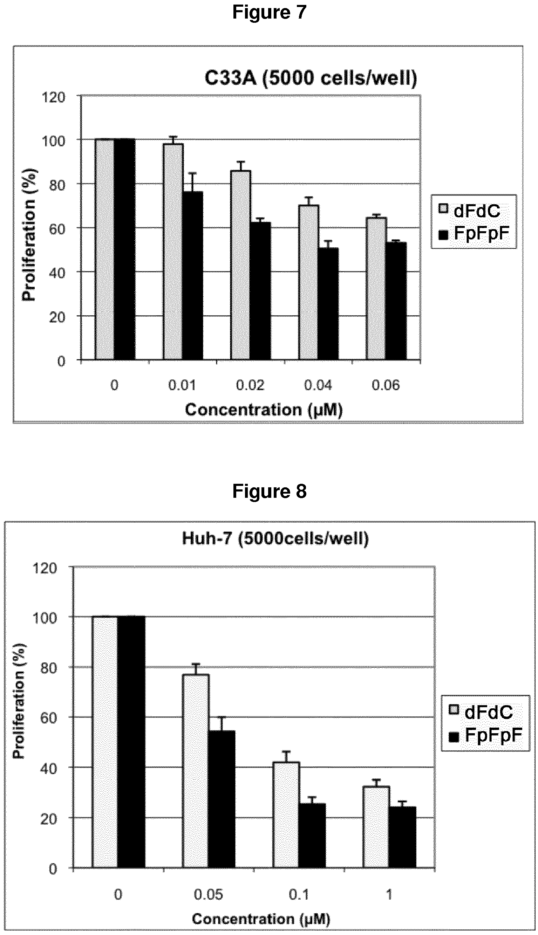

**Figure 7****.** Proliferation of C33A cells untreated (0) or treated with either dFdC or the FpFpF trinucleotide at the indicated concentrations.
**Figure 8****.** Proliferation of Huh-7 cells untreated (0) or treated with either dFdC or FpFpF trinucleotide at the indicated concentrations.

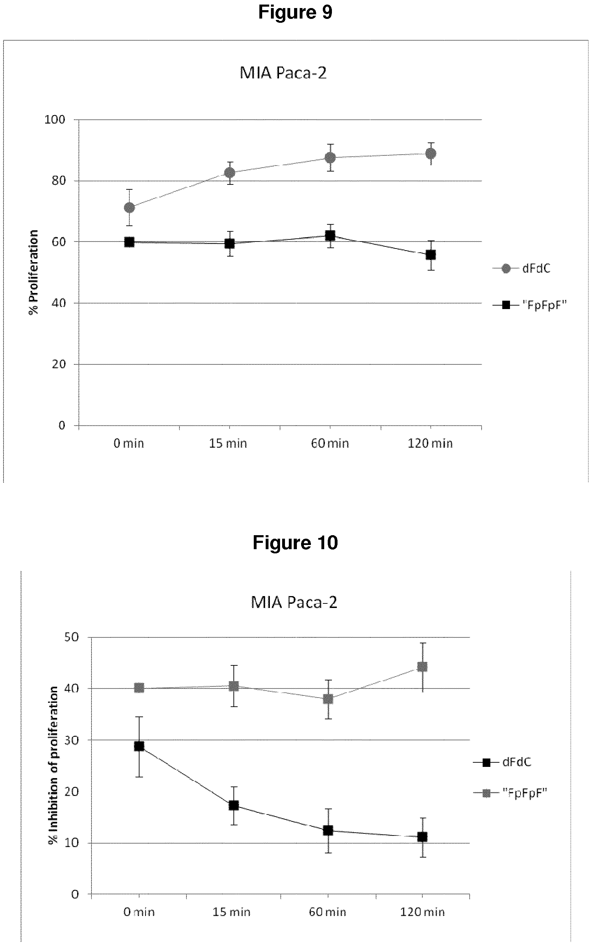

**Figure 9****.** Relative proliferation of MIA PaCa-2 cells treated either with dFdC or FpFpF trinucleotide, which were pre-incubated with human serum the indicated periods of time. Percentage values are relative to those of untreated cells.
**Figure 10****.** Inhibition of proliferation of MIA PaCa-2 cells treated either with dFdC and FpFpF trinucleotide, which were pre-incubated with human serum the indicated periods of time. Percentage values are relative to those of untreated cells.

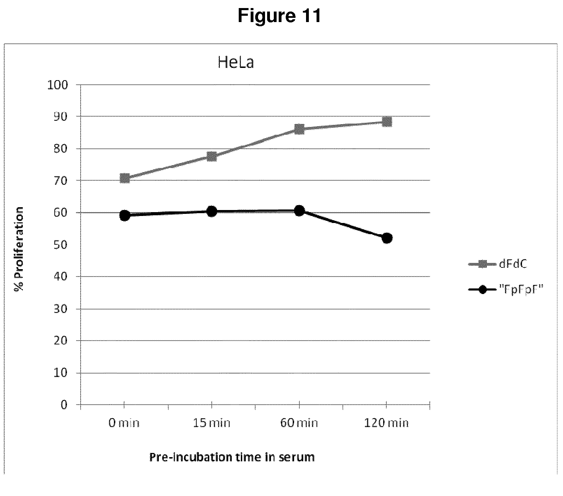

**Figure 11****.** Relative proliferation of HeLa cells treated either with dFdC or FpFpF trinucleotide, which were pre-incubated with human serum the indicated periods of time. Percentage values are relative to those of untreated cells.

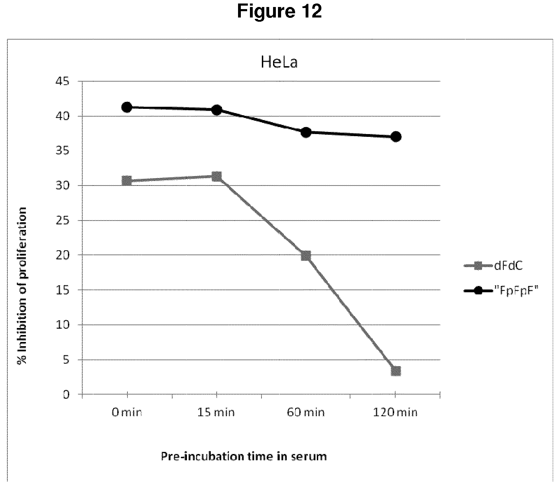

**Figure 12****.** Inhibition of proliferation of HeLa cells treated either with dFdC or FpFpF trinucleotide, which were pre-incubated with human serum the indicated periods of time. Percentage values are relative to those of untreated cells.

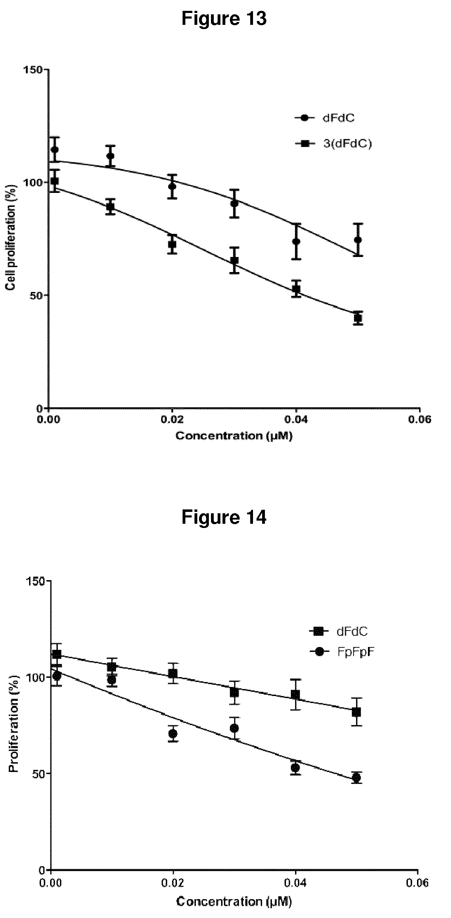

**Figure 13****.** Proliferation of wild-type HeLa cells after treatment with either dFdC or FpFpF trinucleotide at the indicated concentrations.
**Figure 14****.** Proliferation of HeLa cells resistant to dFdC after treatment either with dFdC or FpFpF trinucleotide at the indicated concentrations.
**Figure 15****.** Inhibition of proliferation of Bxpc-3 (A) and MIA PaCa-2 cells (B) treated with either P-dFdC or P-FpFpF, both conjugated with the tumor-binding peptide HBP-1, at the indicated concentrations. Proliferation values refer to untreated cells.

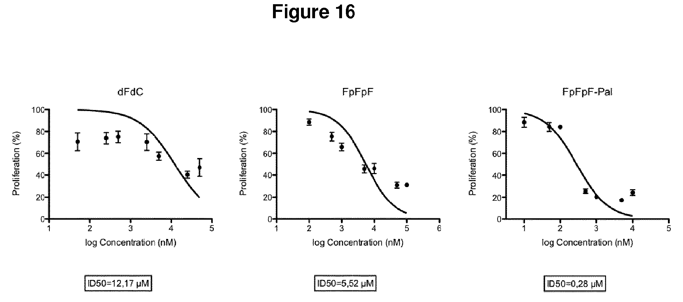

**Figure 16****.** Inhibition of proliferation of Panc-1 cells treated either with dFdC (A), FpFpF (B) or FpFpF-Pal (C) at the indicated concentrations. Proliferation values to untreated cells.

### EXAMPLES

The present invention is described in more detail by the following examples, reference examples and test examples..

### Example 1: Synthesis of the reagents needed for the preparation of oligonucleotides.

Methods and results: The synthesis of the various compounds needed for the production of the oligonucleotides tested in this invention was performed as follows:
***N*⁴-benzoyl-2'-deoxy-2',2'-difluorocytidine (2).** To a suspension of 2'-deoxy-2',2'-difluorocytidine (Gemcitabine, F) (1, C₁₆H₁₅F₂N₂O₅, Carbosynth Limited) (1.5 g, 6.35 mmols) coevaporated in anhydrous pyridine and dissolved in dimethylformamide 3.3 ml (15.8 mmols) of hexamethyldisilazane were added. After stirring for 1 hour at room temperature, the solution was concentrated and the residue was dried over toluene. (Rf: 0.6 20 % MeOH/DCM). The residue was dissolved in anhydrous pyridine and 1.1 ml of benzoyl chloride (9.52 mmol), were added. After 30 minutes of magnetic stirring the solution was concentrated to dryness and then dissolved in toluene and coevaporated. The residue was dissolved in DCM and washed with 1 M sodium bicarbonate and dried over anhydrous MgSO₄ and concentrated to dryness. (Rf. 0.93 7 % MeOH). The removal of trimethysilyl protecting group was carried out by the treatment of the residue with a mixture containing 10 ml of dioxane, 10 ml of methanol and 5 ml of NH₃ (32%) for 15 min at room temperature. The solution was concentrated, dissolved in dichloromethane (DCM) and washed with a 5% of NaHCO₃ and solution saturated of NaCl. Then, the organic phase was concentrated to dryness and the residue was purified by silica gel column chromatography using a gradient of 1% to 4% of methanol (MeOH) in DCM. After concentration of the fractions carrying the desired compound, it was obtained 1.34 g of N⁴-benzoyl-2'-deoxy-2',2'-difluorocytidine **(2).** Yield 62%. C₁₆H₁₅F₂N₃O₅. ¹H NMR(300 MHz, CDCl₃) δ 3.3 (m, 2H, H-5'), 3.8 (dd, 1H, H-4'), 3.9 (m, 2H, H-5'), 4.3 (m, 1H, H-3'), 6.6 (t, 1H, H-1'), 7.6-7.5 (m, 5H, arom-H), 7.9 (m, 1H, H-5), 8.4 (d, 1H, H-6). MS, expected mass M= 367.1; found 368.1 (M+H)⁺.
**5'-*O*-(4,4'-dimethoxytrityl)-*N*⁴-benzoyl-2'-deoxy-2',2'-difluorocytidine** (3). To a solution of 2 (1gr, 2.93 mmols) in anhydrous pyridine 24.4 g (0.2 mmol) of 4-dimethyaminopyridine and 1.09 g (3.2 mmol) of 4,4'-dimethoxytrityl chloride were added. After stirring overnight at room temperature, the reaction was stopped with the addition of 1 ml of MeOH and the resulting solution was concentrated. Then the residue was dissolved in DCM and the organic layer was washed with 5% NaCO₃ aqueous solution and saturated NaCl solution and dried over anhydrous MgSO₄. Purification was accomplished by silica gel column chromatography eluting with DCM to 2% MeOH/DCM obtaining 0.94 g of **3.** Yield 86 %. ¹H-NMR(300 MHz, CDCl₃) δ 3.5 (m, 2H, H-5'), 3.8 (s, 3H, OCH₃), 4.1 (dd, 1H, H-4'), 4.5 (m, 1H, H-3'), 6.5 (m, 1H, H-1'), 6.8-7.8 (m, 18H, arom-H), 8.2 (d, 1H, H-5), 8.6 (m, 1H, H-6). MS expected for C₃₇H₃₃F₂N₃O₇: 669.23. Found: 670.23 (M+H)⁺.
**5'-O-(4,4'-dimethoxytrityl)-3'-O-(2-cyanoethyl-*N*,*N'*-diisopropylphosphoramidite)-N⁴**-**benzoyl-2'-deoxy-2',2'-difluorocytidine (4).** To a solution of **3** (0.5 gr, 0.8 mmols) in anhydrous DCM 0.418 ml (2.4 mmol) of diisopropyethylamine were added together with 0.269 ml (1.2 mmol) of 2-cyanoethyl-*N*,*N*-diisopropylchlorophosphoramidite (1.2 mmol, 268µl) at 0°C under argon atmosphere. After 15 min the reaction was allowed to reach room temperature and stirred for 1 h. The reaction was quenched with washes of 5% NaHCO₃, and saturated NaCl and the organic phase died over *anhydrous* MgSO₄ and concentrated. The residue was purified by silica gel column chromatography (hexanes/EtOAc 1:1 + 2% Et₃N) to give 0.67 g. Yield: 57 % of **4** as a mixture of diastereoisomers. ¹H NMR(300 MHz, CDCl₃) δ 1.0-1.3 (m,12H, 4 CH₃), 2.3 (t, 2H, CH₂CN), 2.6 (t, 2H, CH₂O), 3.5 (m, 2H, H-5'), 3.6 (m, 1H, CH isp), 3.8 (s, 3H, OCH₃), 4.1 (dd, 1H, H-4'), 4.6 (m, 1H, H-3'), 6.5 (m, 1H, H-1'), 6.8-7.8 (m, 18H, arom-H), 8.2 (d, 1H, H-5), 8.63 (m, 1H, H-6), 8.8 (s, 1H, NH). MS expected for C₄₆H₅₀F₂N₅O₈P: 869.34. Found: 870.3 (M+H)⁺.

### Functionalization of the solid support (CPG).

First, compound **3** is converted to the hemissucinate derivative that is loaded to the amino-control pore glass. Compound **3** (20 mg, 0.031 mmol) was dissolved in DCM and treated with N,N.-dimethylaminopyridine (DMAP, 5.75 mg, 0.047 mmol), and succinic anhydride (4.71 mg, 0.047 mmol). The solution was stirred at room temperature overnight. Then the organic phase was washed with 0.1 M NaH₂PO4 and dried over *anhydrous* MgSO₄ and evaporated *in vacuo* to give compound **5** that was used without any further purification.

2,2'-Dithio-bis-(5-nitropyridine) (0.03 mmols, 9.3 mg) dissolved in acetonitrile /dichloroethane (1:1) was mixed with a solution of succinate derivative **5** (20 mg, 0.031 mmol) and DMAP (0.03 mmol, 3.66 mg) dissolved in 500 µl of acetonitrile. To the clear solution obtained 7.8 mg (0.03 mmol) of triphenylphosphine dissolved in 0.1 ml of acetonitrile were added. The mixture was vortexed and added to a vial containing LCA CPG resin (CPG, Inc.) (200 mg, 71 µmol/g). The solid support was collected by filtration and was washed with CH₂Cl₂, CH₃CN, and subsequently dried under vacuum. The solid support was then suspended in 1 ml of acetic anhydride/pyridine/tetrahydrofuran (1:1:8) and 1 ml of solution (10% *N*-methylimidazole in tetrahydrofuran) for 30 minutes. The resulting solid support **(6)** was collected by filtration and washed with MeOH and Et₂O, and dried under vacuum. The loading amount was calculated by trityl cation assay with 70% HClO₄-EtOH to give 38.4 µmol/g.

### Example 2: Synthesis of nucleotide hetero and homo-oligomers containing dFdC.

Methods and results: Oligonucleotides were synthesized using solid-phase phosphoramidite methodology. Syntheses were run on 1 µmol scale on an Applied Biosystems 3400 synthesizer using 5'-*O*-DMT-3'-O-(2-cyanoethyl-*N*,*N'*-diisopropylphosphoramidite-2'-deoxythymidine and phosphoramidite **4.** Controlled-pore glass (CPG) functionalized with 5'-O-DMT-*N*⁴-benzoyl-2'-deoxy-2',2'-difluorocytidine prepared above were used as solid support. The following solutions were used: 0.4 M 1*H*-tetrazole in ACN (activation); 3% trichloroacetic acid in DCM (detritylation), acetic anhydride/pyridine/ tetrahydrofuran (1:1:8) (capping A), 10% N-methylimidazole in tetrahydrofuran (capping B), 0.01 M iodine in tetrahydrofuran/pyridine/water (7:2:1) (phosphate to phosphate oxidation). The coupling time was 15 min for phosphoramidite **4.** All oligonucleotides were synthesized in DMT-ON mode. After the solid-phase synthesis, the solid support was transferred to a screw-cap glass vial and incubated at 55 °C for 1 h with 1.5 mL of NH₃ solution (33%). The vial was then cooled on ice and the supernatant was transferred into a 2 mL eppendorf tube and evaporated to dryness using an evaporating centrifuge. The residue that was obtained was desalted on a NAP-5 column using water as the eluent and evaporated to dryness. The oligonucleotide was purified by HPLC (DMT-ON). Column: Nucleosil 120-10 C₁₈ (250x4 mm); 20 min linear gradient from 15% to 80% B and 5 min 80% B, flow rate 3 mL/min; solution A was 5% ACN in 0.1 M aqueous triethylammonium acetate (TEAA) and B 70% ACN in 0.1 M aqueous TEAA. The pure fractions were combined and evaporated to dryness. The residue that was obtained was treated with 1 mL of 80% AcOH solution and incubated at room temperature for 30 min. The deprotected oligonucleotide was desalted on a NAP-10 column using water as the eluent. All oligonucleotides were quantified by absorption at 260 nm and confirmed by MALDI mass spectrometry. MALDI-TOF spectra were performed using a Perseptive Voyager DETMRP mass spectrometer, equipped with nitrogen laser at 337 nm using a 3ns pulse. The matrix used contained 2,4,6-trihydroxyacetophenone (THAP, 10 mg/mL in ACN/ water 1:1) and ammonium citrate (50 mg/mL in water) or α-cyano-4-hydroxycinnamic acid (ACH 10 mg/mL in ACN/ water 1:1). Dinucleotide TpF: MS expected for C₁₉H₂₃F₂N₅O₁₁P: 566. Found: 566 (M). Dinucleotide FpF: MS expected for C₁₈H₂₉F₄N₆O₁₀P: 587. Found: 587 (M). Trinucleotide FpFpF: MS expected for C₂₇H₃₀F₆N₉O₁₆P₂: 913. Found: 913 (M).

### Example 3: Synthesis of the palmitoyl derivative of the trinucleotide.

The palmitoyl derivative of the trinucleotide was synthesized using solid-phase phosphoramidite methodology as described above. Syntheses were run on 1 µmol scale on an Applied Biosystems 3400 synthesizer using phosphoramidite **4** and the commercially available CPG functionalized with palmitoylamino hexyl 2-deoxyribose (5'-O-(4,4'-dimethoxytrityl)-1-α(6-(palmitoylamino)hexyl)-2-deoxy-D-ribose 3'-O-succinyl-controlled pore glass, Link Technologies) as solid support. After the solid-phase synthesis, the solid support was transferred to a screw-cap glass vial and incubated at room temperature for 4 h with 1.5 mL of NH₃ solution (33%)/ dioxane (1:1). After filtration of the solid support, the supernatant evaporated to dryness. The residue was purified as described above. Palmitoyl-trinucleotide (5'-FpFpF-3'-palmitoyl): MS expected: 1444. Found: 1444 (M).

### Example 4: Cytotoxic activity of dFdC hetero and homo-oligomers.

Objective: Quantify the cytotoxic activity of dFdC hetero and homo-oligomers in cancer cell lines in comparison with dFdC.

Methods: Cervical cancer cell lines (HeLa, SiHa, CaSki and C-33) were cultured at 37°C/5% CO₂ in medium containing DMEM with Glutamax™, 1 mM sodium pyruvate, penicillin/streptomycin (PS) and 10% fetal bovine serum (FBS) (Invitrogen/LifeTechnologies). Pancreatic cancer cell lines were maintained in RPMI 1640 supplemented with Glutamax™, PS and 10% FBS. For the cytotoxicity assay, 96-well plates were plated with 1x10⁴ cells/well. The final volume of culture medium in each well was 100 µL. The cells were treated with different concentrations of drug as indicated. Untreated control cells were set in parallel. The cells were incubated for 72 hours, after which the WST-1 assay (Roche) was performed. This assay utilizes the tetrazolium salt WST-1 [2-(4-Iodophenyl)-3-(4-nitrophenyl)-5-(2,4-disulfophenyl)-2H-tetrazolium], which is cleaved to a soluble formazan in a process that occurs primarily at the cell surface. Because WST-1 reduction is mostly dependent on the glycolytic production of NAD(P)H in viable cells, the amount of formazan dye formed correlates directly with the number of metabolically active cells in the culture. The cells were incubated with WST-1 for 1 hour at 37°C/5% CO₂, then the amount of formazan dye produced was quantified using a scanning spectrophotometer (ELISA plate reader). The absorbance was measured with a test wavelength at 450 nm and a reference wavelength at 630 nm. Then, the 630 nm background absorbance was subtracted from the 450 nm measurement. The mean value of a series of empty wells containing medium but not cells was also subtracted.

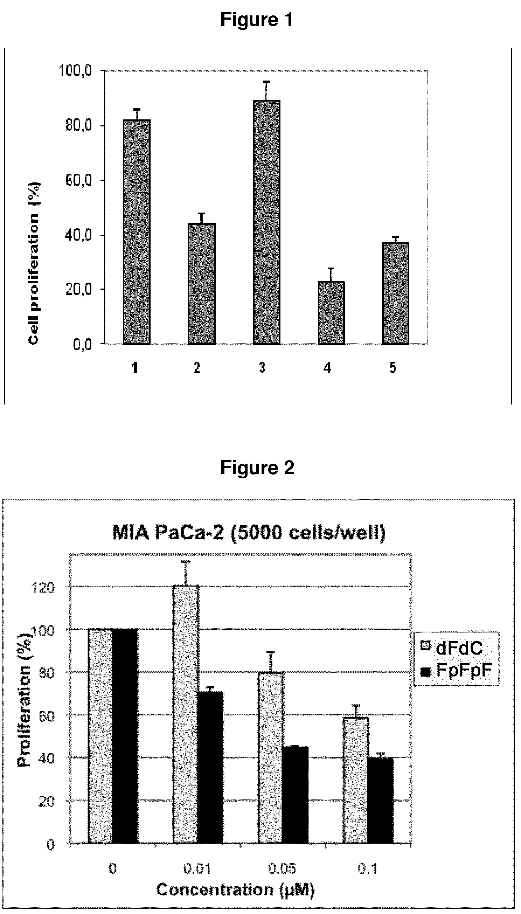

Results:
1) The analysis of the cytotoxic effects of a series of different hetero- and homo-oligomers of dFdC that were tested in the HeLa tumor cell line is illustrated in Figure 1. The tri-homo-oligomer of dFdC (FpFpF trinucleotide) showed a significantly higher rate of inhibition of cell proliferation (∼77%) compared to dFdC (∼63%) (p<0.05) and the di-homo-oligomer FpF (∼56% inhibition) (p<0.03). In addition to the inhibition of cell proliferation the cells treated with these compounds showed cytopathic effects (changes in morphology to a rounded shape, vacuolization and others). Under the same conditions, hetero-oligomers, such as TpF dinucleotide and TpFpF trinucleotide were less active in inhibiting cell growth (19% and 11% inhibition, respectively).
2) The FpFpF trinucleotide was tested in comparison with dFdC for its ability to inhibit cell growth of tumor cells of different origin: pancreatic cancer (MIA PaCa-2, ASPC) cervical (HeLa, CaSki, C33A) and hepatic (Huh-7) cancers. In all cases the cells were plated the day before treatment to a density of 5000 cells/well in 96-multiwell plates. The cells (6 wells per condition) were treated at the concentrations indicated and incubated for further 72 hours. At this time cell proliferation was quantified using the WST-1 assay as described above. Control wells with untreated cells and without cells were run in parallel. The values obtained with the later were subtracted from all others. Mean values corresponding to wells that were not treated were set to 100% proliferation and the mean values obtained with the treated cells were referred to them. As illustrated in the figures C to I, under these conditions in all cases the FpFpF trinucleotide inhibited cell proliferation with significantly higher efficiency than the dFdC monomer.

### Example 5: Stability of FpFpF trinucleotide in human serum.

Objective: To demonstrate a higher stability of the FpFpF trinucleotide in human serum compared to dFdC.

Methods: The cells were seeded in 96-well plates one day before treatment at a density of 5000 cells/well in 100 µl/well of medium DMEM/Glutamax™/1 mM sodium pyruvate/PS/10%/FBS and cultured at 37°C/5% CO₂. Separate serum dilutions of dFdC and FpFpF trinucleotide were prepared from 1 mM stock solutions to a final concentration of 20 µmol/L. The sera aliquots containing either drug were incubated at 37°C/5% CO₂ for the indicated times before they were added to the cells to the indicated final concentrations. The cells were incubated for further 72 hours after which the cell proliferation was quantified using the WST-1 assay. Two controls were run in parallel: untreated cells and cells treated with dFdC or FpFpF trinucleotides that were not incubated with serum.

Results: The data obtained with the MIA PaCa-2 and HeLa cell lines are shown in the figures J-K and L-M, respectively. These results obtained with both cell lines show that FpFpF is stable in human serum for at least 2 hours. The inhibition of proliferation with FpFpF in this period remained at about 40%, while the inhibition with dFdC decayed to 5% (HeLa) and 10% (MIA PaCa-2) in the same period of time.

### Example 6: Cytotoxic activity of FpFpF trinucleotide on cancer cells resistant to dFdC

Objective: To demonstrate the anti-proliferative activity of FpFpF trinucleotide in tumor cells with resistance to dFdC

Methods: Wild-type (parental) HeLa cells were sequentially treated with four rounds of increasing concentrations (5 to 40 nM) of dFdC until they became resistant to the drug and were able to proliferate after treatment with dFdC at the highest concentration. The parental and resistant cells were plated one day before treatment at a density of 5000 cells/well in 96-well plates with 100 µl/well of medium DMEM/Glutamax™/1 mM sodium pyruvate/PS/10%FBS and cultured at 37°C/5% CO₂. Treatments (6 wells per condition) with wither dFdC or FpFpF were carried out at the concentrations specified in the figures N and O, and the cells were incubated for 72 hours after which proliferation was measured using the WST-1 assay. Untreated cells were used as reference to set the 100% proliferation values. Results. HeLa cells resistant to dFdC were significantly sensitive to the anti-proliferative effects of FpFpF (compare Figures N and O).

### Example 7: Cytotoxic activity of FpFpF trinucleotide bound to peptide

Objective: To prove and quantify the inhibition of cell proliferation caused by FpFpF trinucleotide conjugated to a peptide that specifically binds tumor cells

Methods: Targeted therapies are intended to enhance accumulation of chemotherapy drugs into tumor cells thereby causing a more potent cytotoxic effect. One way to achieve this is using peptides binding surface proteins on tumor cells with high affinity. One such peptide, H₂N-SPRGDLAVLGHKY-COOH (HBP-1) (SEQ ID No: 1) (Nothelfer et al, 2009), has been previously shown to bind tumor cells via the intrinsic RGD motif. This peptide was synthesized, purified and conjugated to FpFpF or dFdC using the water-soluble carbodiimide cross-linker EDC (Thermo Scientific Pierce) following the manufacturer instructions. Excess reagent and cross-linking byproducts were removed by successive passes through two Sephadex G-25 columns. The concentration of peptide in the cross-linked product was determined by the BCA method (Thermo Scientific Pierce) and the concentration of dFdC or FpFpF bound to peptide in the purified conjugates (abbreviated P-dFdC and P-FpFpF, respectively) was measured spectrophotometrically (A260 nm) against standard curves.

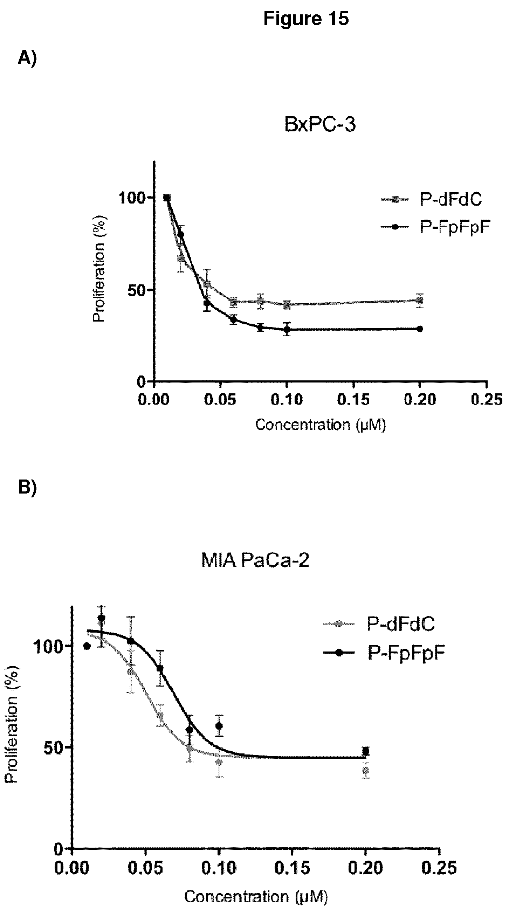

Results. The effects of P-dFdC and P-FpFpF on proliferation of the pancreatic cancer cell lines BxPC-3 and MIA PaCa-2 are shown in the figure 15. Treatment with P-FpFpF caused a higher inhibition of cell proliferation compared to treatment with P-dFdC. The two products are likely to enter the cells by interacting with integrins, such as the αvβ6 integrin, through the RGD motif. The pancreatic cancer cell line BxPC-3 expresses high levels of avb6, whereas MIA PaCa-2 cells do not express αvβ6 (Hausner et al, 2009). The WST-1 assay showed that BxPC-3 cells the IC50 for P-dFdC was 0.17 µM and for P-FpFpF was 0.05 µM. In turn, with MIA PaCa-2 cells the inhibition by P-FpFpF was lower than that by P-dFdC: the IC50 for P-dFdC was 0.078 µM and for P-FpFpF was 0.105 µM. The data show that conjugation of FpFpF with an αvβ6-binding peptide enhances its cell growth inhibitory properties in pancreatic cancer cells expressing the αvβ6 integrin.

### Example 8: Cytotoxic activity of FpFpF trinucleotide coupled to lipid

Objective: Improve the cell permeability and activity of FpFpF in tumor cells

Methods: The FpFpF trinucleotide was coupled to lipid as described in the Example 3 for the Palmitoyl-trinucleotide (5'-FpFpF-3'-Pal). Panc-1 cells were plated one day before treatment at a density of 5000 cells/well in a 96-well plate with 100 µl/well of medium RPMI/Glutamax™/1 mM sodium pyruvate/PS/10%FBS and cultured at 37°C/5% CO₂. Treatments (6 wells per condition) with wither dFdC, FpFpF and FpFpF-Pal were carried out at the indicated concentrations.

Results: The FpFpF-Pal derivative exhibited the highest inhibitory power with an ID50 of 0.28 µM compared to 12.17 and 5.52 µM for dFdC and FpFpF, respectively.

### Example 9: Encapsulation of FpFpF trinucleotide into liposomes

Objective: To improve FpFpF trinucleotide delivery through liposome encapsulation Methods. Naked or antibody-targeted liposomes (LPs) carrying anticancer compounds may increase the therapeutic efficacy of these agents. The lipids were hydrated in saline solution for two hours at room temperature (LPs). Then, the reconstituted LPs were diluted with 1 ml of saline solution and applied to Amicon Ultra-4 NMWL100 kDa centrifugal filters (Millipore) and washed three times with 1 ml of saline solution. Maleimido-activated phospholipids are suitable for reaction with sulfhydryl groups on antibodies. For conjugation of anti-Muc1 antibodies to liposomes, antibody was added to a final concentration of 40 µg/ml (Ab-LPs). The mixture was incubated at ambient temperature for two hours. MCF-7 cells (10⁶ cells/ml) were incubated in medium RPMI with either 1) gemcitabine, 2) dFdC homo-oligomers, or 3) 1 mg(LP)/ml Ab-LPs. After 30 minutes equal aliquots of the cells were centrifuged and washed twice with RPMI medium.

Results: Compared with unencapsulated FpFpF, the FpFpF trinucleotide showed higher inhibitory activity on proliferation of the Panc-1 and BxPc-3 cell lines.

### Example 10: Anti-proliferative activity of FpFpF trinucleotide in vivo

Objective: To compare the anti-tumor activities of FpFpF trinucleotide and dFdC in vivo in a xenograft model. HeLa cells and immunodeficient mice were used in this assay as described below.

Methods. Immunodeficient CAnN.Cg-*Foxnl^{nu}*/Crl (BALB/c) mice (The Jackson Laboratories) are well suited for the xenotransplantation of human tumors. HeLa cells (5 x 10⁶) were mixed 1:1 with matrigel in a final volume of 0.1 ml and then injected subcutaneously in the right flank of each mouse. Treatments were initiated at the time when the tumor volume reached approximately 200 mm³. The drugs (FpFpF trinucleotide or dFdC) were administered intraperitoneally at 10 mg/kg every fourth day for a total of four doses. Tumor volumes were calculated with the ellipsoid volume formula (L x W x H x π/6) (Tomayko and Reynolds, 1989). Tumor volume values were transformed to a log scale. A two-way analysis of variance by time and treatment was used. The data are finally expressed as means and standard errors for each treatment group against time. A Weibull distribution was assumed to calculate mean times and standard errors for each treatment group. Tumor growth delay was defined as the difference in mean time between each treated group and the control group.

Results. Data in Table 1 show the effects of FpFpF trinucleotide in comparison with dFdC. Treatment with FpFpF trinucleotide caused a statistically significant delay (p<0.01) in tumor growth compared to dFdC.

**Table 1. Tumor Growth Delay at different tumor sizes.**

| Treatment group | 500 mm³ Mean ± S.E.M. (days) | 700 mm³ Mean ± S.E.M. (days) | 900 mm³ Mean ± S.E.M. (days) |
|---|---|---|---|
| Saline | 0.0±0.8 | 0.0±0.7 | 0.0±0.7 |
| dFdC | 2.8±0.3 | 4.9±0.6 | 7.7±0.5 |
| FpFpF trinucleotide | 14.8±0.7 | 17.7±0.5 | 27.3±0.8 |

### REFERENCES

Abbruzzese et al 1991, J Clin Oncol 9:491-498
Bornmann et al 2008, Cancer Chemother Pharmacol 61:395-405
Davidson et al 2004, Cancer Res 64: 3761-3766
Gmeiner et al (1995) US Patent Number 5,457,187
Hausner et al 2009, Cancer Res 69: 5843-5850
Liu et al 1999, Nucleosides & Nucleotides, 18 (8): 1789-1802
Moog et al 2000, Int J Pharm 206:43-53
Moog et al 2002, Cancer Chemother Pharmacol 49: 356-66
Nakahira et al 2007, Int J Cancer 120: 1355-1363
Noble and Goa 1997. Drugs 54:447-72
Nothelfer et al 2009, J Nuclear Medicine 50: 426-434
Reid et al 2004, J Clin Oncol 22: 2445-2451
Sampath et al (2010) US Patent Application US 2010/0081627
Shipley et al 1992, Drug Metab Dispos 20: 849-855
Tomayko and Reynolds 1989, Cancer Chemother Pharmacol 24: 148-154

## Claims

1. Compound made of 2-30 units of 2',2'-difluoro-2'-deoxycytidine (dFdC) linked with 5' -3' phosphate bonds for use in therapy

2. Compound made of 2-30 units of dFdC linked with 5' -3' phosphate bonds for use in treating an infectious disease or cancer.

3. The compound for the use of claim 1 or 2, which is made of 2-10 units of dFdC linked with 5' -3' phosphate bonds.

4. The compound for the use of any one of claims 1 to 3, which is FpFpF (dFdC trinucleotide) linked with 5' -3' phosphate bonds.

5. The compound for the use of any one of claims 1-4 with a covalently linked molecule of any of the groups consisting of lipid, amino acid, peptide, antibody or aptamer at the 5' - or 3' -terminus, or at both termini.

6. The compound for the use of any one of claims 1-4 which is encapsulated in a liposome.

7. The compound for the use of any one of claims 1-6, wherein said compound is modified to increase its stability in serum and cell permeability, for example by conjugation with lipid.

8. The compound for the use of any one of claims 1-7, wherein said use comprises an administration of said compound simultaneously with dFdC.

9. The compound for the use of any one of claims 1-7, wherein said use comprises an administration of said compound and dFdC hydrochloride separately, in any order, within a therapeutically effective interval.

10. The compound for the use of claim 8 or 9, wherein said administration is via the parenteral route.

11. The compound for the use of any one of claims 2-10, wherein said cancer is pancreatic cancer, hepatocellular cancer, lung cancer, cervical precancer and cancer lesions, breast cancer, ovarian cancer, colorectal cancer or lymphoma, such as non-Hodgkin' s lymphoma.

12. The compound for the use of any one of claims 1-11, wherein said compound is homomeric.

13. The compound according to any one of claims 1-12 for the use of any one of claims 1-12 having structural Formula (I) 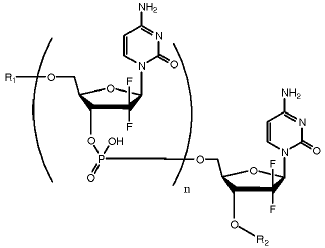 or a pharmaceutically acceptable salt, hydrate, N-oxide or solvate thereof, wherein:
R₁ is hydrogen, lipid, amino acid, peptide, antibody or aptamer
R₂ is hydrogen, lipid, amino acid, peptide, antibody or aptamer n= 1-30

14. A composition comprising the compound for use as defined in any one of claims 1-13.

15. The composition of claim 14 which is a pharmaceutical composition.

## Patentansprüche

1. Verbindung, hergestellt aus 2-30 Einheiten von 2',2'-Difluor-2'-deoxycytidin (dFdC) verbunden mit 5'-3'-Phosphatbindungen zur Verwendung in der Therapie.

2. Verbindung, hergestellt aus 2-30 Einheiten von dFdC verbunden mit 5'-3'-Phosphatbindungen zur Verwendung in der Behandlung einer Infektionskrankheit oder Krebs.

3. Die Verbindung zur Verwendung nach Anspruch 1 oder 2, welche aus 2-10 Einheiten von dFdC verbunden mit 5'-3'-Phosphatbindungen hergestellt ist.

4. Die Verbindung zur Verwendung nach einem der Ansprüche 1 bis 3, welche FpFpF (dFdC-Trinukleotid) verbunden mit 5'-3'-Phosphatbindungen ist.

5. Die Verbindung zur Verwendung nach einem der Ansprüche 1-4 mit einem kovalent am 5'-oder 3'-Terminus oder an beiden Termini verbundenen Molekül aus einer der Gruppen, bestehend aus Lipid, Aminosäure, Peptid, Antikörper oder Aptamer.

6. Die Verbindung zur Verwendung nach einem der Ansprüche 1-4, welche in ein Liposom verkapselt ist.

7. Die Verbindung zur Verwendung nach einem der Ansprüche 1-6, wobei die Verbindung modifiziert ist zur Erhöhung der Stabilität im Serum und Zellpermeabilität, beispielsweise durch Konjugation mit einem Lipid.

8. Die Verbindung zur Verwendung nach einem der Ansprüche 1-7, wobei die Verwendung die Verabreichung der Verbindung gleichzeitig mit dFdC umfasst.

9. Die Verbindung zur Verwendung nach einem der Ansprüche 1-7, wobei die Verwendung eine getrennte Verabreichung der Verbindung und dFdC Hydrochlorid in beliebiger Reihenfolge umfasst innerhalb eines therapeutisch wirksamen Intervalls.

10. Die Verbindung zur Verwendung nach Anspruch 8 oder 9, wobei es sich bei der Verabreichung um eine parenterale Verabreichung handelt.

11. Die Verbindung zur Verwendung nach einem der Ansprüche 2-10, wobei der Krebs Bauchspeicheldrüsenkrebs, Hepatuzelluärer Krebs, Lungenkrebs, Zervicaltumor oder -tumorvorstufe, Brustkrebs, Ovarialkarzinom, Dickdarmkrebs oder -lymphom, wie das Non-Hodgkin' Lymphom ist.

12. Die Verbindung zur Verwendung nach einem der Ansprüche 1-11, wobei die Verbindung ein Homomer ist.

13. Die Verbindung nach einem der Ansprüche 1-12 zur Verwendung nach einem der Ansprüche 1-12 mit der Strukturformel 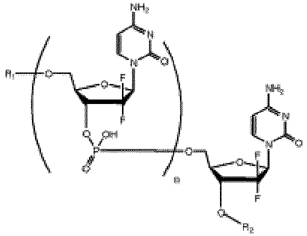 oder eine pharmazeutische verträgliches Salz, Hydrat, Stickoxid oder eine Lösung davon, wobei:
R₁ ein Wasserstoff, ein Lipid, eine Aminosäure, ein Peptid, ein Antikörper oder ein Aptamer ist
R₂ ein Wasserstoff, ein Lipid, eine Aminosäure, ein Peptid, ein Antikörper oder ein Aptamer mit n = 1-30 ist.

14. Eine Zusammensetzung, die die Verbindung zur Verwendung wie in einem der Ansprüche 1-13 definiert enthält.

15. Die Zusammensetzung nach Anspruch 14, welche eine pharmazeutische Zusammensetzung ist.

## Revendications

1. Composé constitué de 2-30 unités de 2',2'-difluoro-2'-désoxycytidine (dFdC) liées par des liaisons phosphate 5'-3' destiné à être utilisé dans une thérapie.

2. Composé constitué de 2-30 unités de dFdC liées par des liaisons phosphate 5'-3' destiné à être utilisé dans le traitement d'une maladie infectieuse ou du cancer.

3. Composé destiné à être utilisé selon la revendication 1 ou 2, qui est constitué de 2-10 unités de dFdC liées par des liaisons phosphate 5'-3'.

4. Composé destiné à être utilisé selon l'une quelconque des revendications 1 à 3, qui est la FpFpF (trinucléotide de dFdC) liée par des liaisons phosphate 5'-3'.

5. Composé destiné à être utilisé selon l'une quelconque des revendications 1 à 4 avec une molécule liée de façon covalente de l'un quelconque des groupes consistant un lipide, un acide aminé, un peptide, un anticorps ou un aptamère, à la terminaison 5' ou 3', ou aux deux terminaisons.

6. Composé destiné à être utilisé selon l'une quelconque des revendications 1 à 4 qui est encapsulé dans un liposome.

7. Composé destiné à être utilisé selon l'une quelconque des revendications 1 à 6, où ledit composé est modifié pour augmenter sa stabilité dans le sérum et sa perméabilité cellulaire, par exemple par une conjugaison avec un lipide.

8. Composé destiné à être utilisé selon l'une quelconque des revendications 1 à 7, où ladite utilisation comprend une administration dudit composé simultanément avec de la dFdC.

9. Composé destiné à être utilisé selon l'une quelconque des revendications 1 à 7, où ladite utilisation comprend une administration dudit composé et de chlorhydrate de dFdC séparément, dans un ordre quelconque, dans un intervalle thérapeutiquement efficace.

10. Composé destiné à être utilisé selon la revendication 8 ou 9, où ladite administration est via la voie parentérale.

11. Composé destiné à être utilisé selon l'une quelconque des revendications 2 à 10, où ledit cancer est le cancer du pancréas, le cancer hépatocellulaire, le cancer du poumon, le pré-cancer et des lésions cancéreuses du col de l'utérus, le cancer du sein, le cancer de l'ovaire, le cancer colorectal ou un lymphome, comme un lymphome non Hodgkinien.

12. Composé destiné à être utilisé selon l'une quelconque des revendications 1 à 11, où ledit composé est homomérique.

13. Composé selon l'une quelconque des revendications 1 à 12 destiné à être utilisé selon l'une quelconque des revendications 1 à 12, possédant la formule structurale (I) 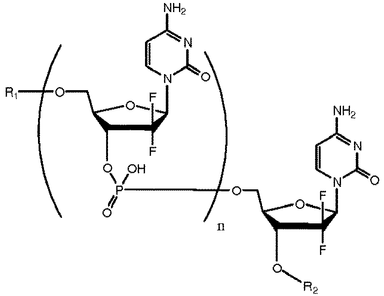 ou un sel, hydrate, N-oxyde ou solvate de celui-ci pharmaceutiquement acceptable, dans laquelle :
R₁ est un hydrogène, un lipide, un acide aminé, un peptide, un anticorps ou un aptamère
R₂ est un hydrogène, un lipide, un acide aminé, un peptide, un anticorps ou un aptamère
n = 1-30.

14. Composition comprenant le composé destiné à être utilisé tel que défini dans l'une quelconque des revendications 1 à 13.

15. Composition selon la revendication 14, qui est une composition pharmaceutique.
